# EUROPEAN PATENT APPLICATION

(11) **EP 2 950 133 A1**
(43) Date of publication of application: **02.12.2015**
(21) Application number: 15169590.5
(22) Date of filing: 28.05.2015
(51) Int. Cl.: G02B 27/01, G06F 3/01

(54) **DISPLAY SYSTEM, DISPLAY APPARATUS, DISPLAY CONTROL APPARATUS, DISPLAY METHOD, AND PROGRAM**

(30) Priority: 29.05.2014 JP 2014110947
(71) Applicant: Seiko Epson Corporation, Tokyo 163-0811 (JP)
(72) Inventor: Tanaka, Hideki, Nagano, 392-8502 (JP)
(74) Representative: Hoffmann Eitle

(57) **Abstract**

A display system has a display apparatus configured to be attached to a head of the user, and a display control apparatus connected to the display apparatus. The display control apparatus has a detection part that detects a tilt of the user, and a transmitting part that transmits a first tilt image representing the tilt to the display apparatus, and the display apparatus has a receiving part that receives the first tilt image from the display control apparatus, and a display part that displays the first tilt image.

## Description

### BACKGROUND

### 1. Technical Field

The present invention relates to a display system, a display apparatus, a display control apparatus, a display method, and a program.

### 2. Related Art

Head mounted displays worn and used on heads of users are known. Of these head mounted displays, there are transmissive (also referred to as "see-through") head mounted displays by which the user may see the outside world while wearing the display.

For example, JP-A-11-85051 discloses a head mounted display housing a display screen unit in a main body. The display screen unit is rotatable with respect to the main body.

After brain disorders such as strokes, abnormalities in posture and balance may arise due to various factors. One postural adjustment disorder is known as the pusher syndrome. To improve quality of life while living with this disorder, rehabilitation (also referred to as "rehab") is important. In the rehab, for example, a patient is instructed to stand according to a vertical reference and a caregiver assists the patient.

However, a patient afflicted with the above described disorder may be unaware of a posture that is tilted. In this case, if the patient is simply instructed to stand according to the vertical reference, the rehab is unsuccessful. Further, in the rehab, visual recognition of the posture by the patient himself or herself is effective, however, it is not easy for the patient to undergo rehab because preparation of a large mirror is required for this observation and it is also difficult for the patient to face the mirror.

### SUMMARY

An advantage of some aspects of the invention is to improve the rehabilitation of a patient having an abnormality in posture and balance.

A first aspect of the invention is directed to a display system including a display apparatus configured to be attached to a head of the user, and a display control apparatus connected to the display apparatus, the display control apparatus includes a detection part that detects a tilt of the user, and a transmitting part that transmits a first tilt image representing the tilt to the display apparatus, and the display apparatus includes a receiving part that receives the first tilt image from the display control apparatus, and a display part that displays the first tilt image. With this configuration, the tilt of the body may be easily recognized and the rehab may be easily performed.

In the display system, the display control apparatus may include a first sensor that detects the tilt and may be attached to the user. With this configuration, the rehab may be supported by the simple system configuration of the two of the display apparatus and the display control apparatus.

In the display system, the display system may further include a second sensor connected to the display control apparatus, and the second sensor may be attached to the user, the detection part may detect tilts of one or more locations of the user by the first sensor and the second sensor, the transmitting part may transmit a second tilt image representing the tilts of the one or more locations to the display apparatus, the receiving part may receive the second tilt image from the display control apparatus, and the display part may display the second tilt image. With this configuration, for example, images representing tilts of a plurality of locations of the body are displayed by addition of the second sensor to the system, and the posture of him- or herself may be recognized more easily and accurately.

In the display system, the display system may further include a second sensor connected to the display control apparatus, and the second sensor may be attached to the user, the detection part may detect tilts of one or more locations of the user by the second sensor, the transmitting part may transmit a third tilt image representing the tilts of the one or more locations to the display apparatus, the receiving part may receive the third tilt image from the display control apparatus, and the display part may display the third tilt image. With this configuration, for example, it is unnecessary to attach the display control apparatus to the body of the user, and the caregiver or the patient may easily hold and operate the display control apparatus in the hand. Further, for example, images representing tilts of a plurality of locations of the body are displayed by addition of the second sensor to the system, and the posture of him- or herself may be recognized more easily and accurately.

In the display system, the first tilt image may include an image representing a tilt of a torso of the user. With this configuration, for example, for a patient having a symptom that tilts the head and the torso oppositely, the tilt of the torso may be recognized more easily.

A second aspect of the invention is directed to a display system including a display apparatus configured to be attached to a head of a user, a display control apparatus connected to the display apparatus, an information processing apparatus connected to the display control apparatus, and a second sensor attached to the user, the information processing apparatus includes a detection part that detects tilts of one or more locations of the user by the second sensor, and a transmitting part that transmits a fourth tilt image representing the tilts of the one or more locations to the display control apparatus, the display control apparatus includes a receiving part that receives the fourth tilt image from the information processing apparatus, and a transmitting part that transmits the fourth tilt image to the display apparatus, and the display apparatus has a receiving part that receives the fourth tilt image from the display control apparatus, and a display part that displays the fourth tilt image. With this configuration, the tilt of the body may be easily recognized and the rehab may be easily performed. Further, for example, load of image processing etc. in the display control apparatus may be reduced.

In the display system, the fourth tilt image may include an image representing a tilt of a torso of the user. With this configuration, for example, for a patient having a symptom that tilts the head and the torso oppositely, the tilt of the torso may be recognized more easily.

In the display system, the display part may further display an image representing a vertical direction or a horizontal direction. With this configuration, for example, the tilt of the body may be recognized with a relative relationship with the vertical direction or the horizontal direction, and the tilt of the body may be easily recognized and the rehab may be easily performed.

In the display system, at least one of the display apparatus and the display control apparatus may have a sound output part that outputs an alarm when a tilt of a torso of the user is beyond a predetermined range. With this configuration, for example, the rehab may be supported while attention is drawn.

A third aspect of the invention is directed to a display apparatus configured to be attached to a head of a user including a display part that displays a first tilt image representing a tilt of the user. With this configuration, the tilt of the body may be easily recognized and the rehab may be easily performed.

In the display apparatus, a receiving part that receives the first tilt image from a display control apparatus may be provided. With this configuration, for example, the tilt of the user may be detected by the display control apparatus attached to another location than the head of the user and displayed on the display apparatus.

In the display apparatus, the first tilt image may include an image representing a tilt of a torso of the user. With this configuration, for example, for a patient having a symptom that tilts the head and the torso oppositely, the tilt of the torso may be recognized more easily.

A fourth aspect of the invention is directed to a display control apparatus connected to a display apparatus configured to be attached to a head of a user including a detection part that detects a tilt of the user, and a transmitting part that transmits a first tilt image representing the tilt to the display apparatus. With this configuration, for example, the tilt of the body is displayed on the display apparatus, and the tilt of the body may be easily recognized and the rehab may be easily performed.

In the display control apparatus, the first tilt image may include an image representing a tilt of a torso of the user. With this configuration, for example, the tilt of the torso is displayed on the display apparatus, and, for a patient having a symptom that tilts the head and the torso oppositely, the tilt of the torso may be recognized more easily.

A fifth aspect of the invention is directed to a display method in a display system having a display apparatus configured to be attached to a head of a user including detecting a tilt of the user, generating a first tilt image representing the tilt, and displaying the first tilt image. With this configuration, the tilt of the body may be easily recognized and the rehab may be easily performed.

A sixth aspect of the invention is directed to a program of a display control apparatus connected to a display apparatus configured to be attached to a head of a user, allowing the display control apparatus to execute detecting a tilt of the user, and transmitting a first tilt image representing the tilt to the display apparatus. With this configuration, for example, the tilt of the body is displayed on the display apparatus, and the tilt of the body may be easily recognized and the rehab may be easily performed.

### BRIEF DESCRIPTION OF THE DRAWINGS

Embodiments of the invention will be described with reference to the accompanying drawings, wherein like numbers reference like elements.
Fig. 1 shows an example of an appearance of a display system according to a first embodiment of the invention.
Fig. 2 is a diagram for explanation of an outline of a display apparatus.
Fig. 3 is a diagram for explanation of an example of attachment locations of the display apparatus etc. to a user.
Fig. 4 shows an example of a configuration of the display system.
Fig. 5 is a flowchart showing an example of an operation of the display system.
Figs. 6A to 6D show examples of images representing tilts.
Fig. 7 shows a screen example displayed on the display apparatus.
Fig. 8 is a diagram for explanation of an example of attachment locations of a display apparatus etc. according to a second embodiment of the invention to a user.
Fig. 9 shows an example of a configuration of a display system.
Fig. 10 is a flowchart showing an example of an operation of the display system.
Fig. 11 is a diagram for explanation of an example of attachment locations of a display apparatus etc. according to a third embodiment of the invention to a user.
Fig. 12 shows an example of a configuration of a display system.
Fig. 13 shows an example of an operation screen according to a first modified example.
Fig. 14 shows an example of an image representing a tilt.
Figs. 15A and 15B are diagrams for explanation of an example of a symptom of the pusher syndrome.

### DESCRIPTION OF EXEMPLARY EMBODIMENTS

First, for understanding of the invention, the pusher syndrome is explained as an example of an abnormality in posture and balance.

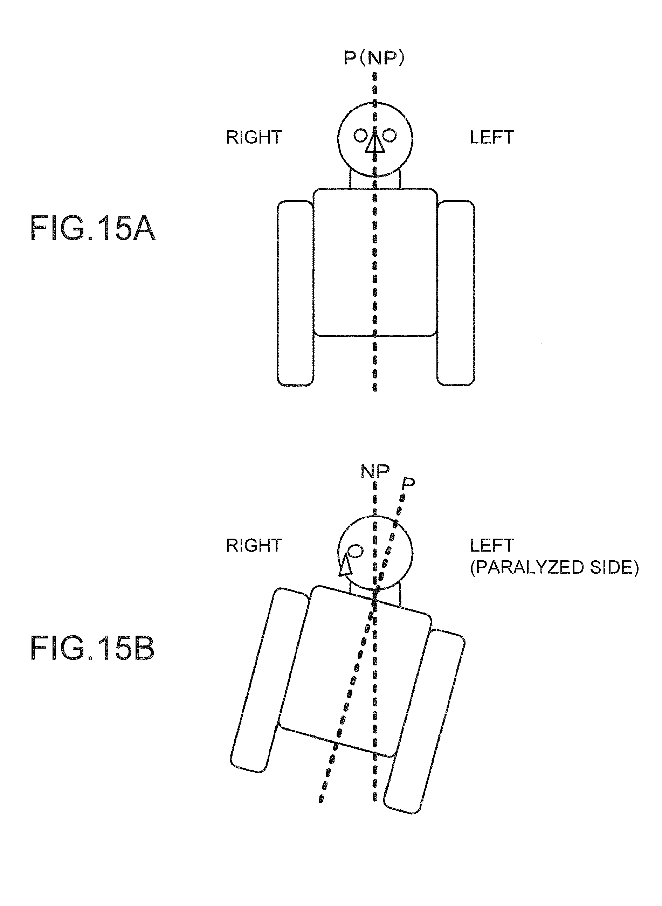

Figs. 15A and 15B are diagrams for explanation of an example of a symptom of the pusher syndrome. Fig. 15A shows a posture in a normal case (without the pusher syndrome) and Fig. 15B shows a posture in a case of the pusher syndrome. Figs. 15A and 15B are front views of upper bodies including torsos of patients. The broken lines in the drawings show body axes of the torsos.

As shown in Fig. 15A, the normal patient has a body axis P along the median line (i.e., aligned with the normal body axis NP) and a face and a torso facing the front. On the other hand, as shown in Fig. 15B, in the patient of the pusher syndrome, a symptom of a tilt of the body axis P toward the paralyzed side, in some cases, the face facing the non-paralyzed side appears.

For a rehab of the patient, visual recognition of the posture of him- or herself is effective. However, in the case of the condition shown in Fig. 15B, if a vertical reference (e.g., a bar showing the body axis NP or the like) is placed in front of the patient, it is difficult for the patient to see the vertical reference. Further, if a mirror is placed in front of the patient, it is difficult for the patient to see the reflection in the mirror. Accordingly, it is difficult for a skilled caregiver to proceed with the rehab smoothly.

In embodiments of the invention, as will be explained in the following embodiments, a current body axis of a patient is displayed on a display apparatus.

### First Embodiment

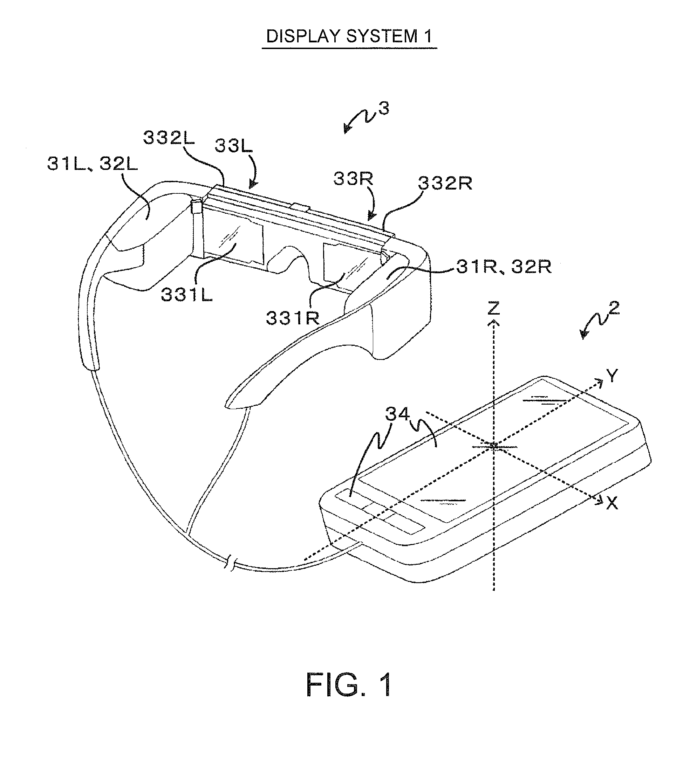

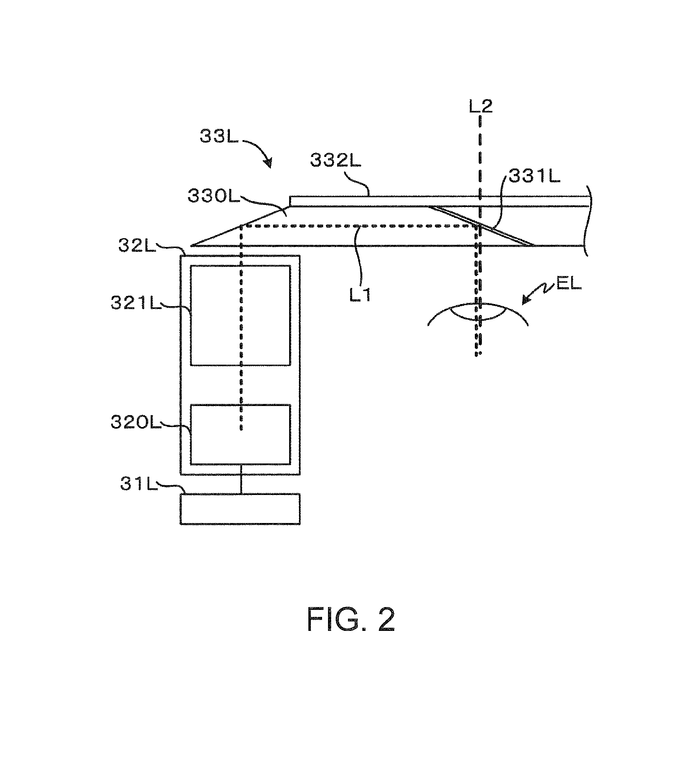

Fig. 1 shows an example of an appearance of a display system according to the first embodiment of the invention. Fig. 2 is a diagram for explanation of an outline of a display apparatus.

A display system 1 has a controller 2 and a display apparatus 3. The controller 2 and the display apparatus 3 have a wired connection that enables communication with each other. Alternatively, the controller 2 and the display apparatus 3 may have a wireless connection. The controller 2 corresponds to a display control apparatus.

The controller 2 is e.g. a portable computer or the like and a terminal device for controlling the display apparatus 3. The controller 2 stores contents including an image of a display object, generates image signals based on the contents, and transmits the image signals to the display apparatus 3 for display. Note that, in the embodiment, as shown in Fig. 1, the controller 2 is seen in a plan view, and an X-axis along the horizontal direction, a Y-axis along the vertical direction, and a Z-axis orthogonal to the X-axis and the Y-axis are set.

The display apparatus 3 is a spectacle-shaped transmissive head mounted display. For example, the display apparatus 3 performs projection on screens provided for right and left eyes (transmissive screens that transmit light of the outside world) based on the image signals received from the controller 2, and thereby, displays images to a user.

Specifically, a configuration with respect to the display of the display apparatus 3 will be explained. The display apparatus 3 includes display control parts 31 (left display control part 31L, right display control part 31R), projection parts 32 (left projection part 32L, right projection part 32R), and display parts 33 (left display part 33L, right display part 33R). The left display control part 31L for left eye and the right display control part 31R for right eye, the left projection part 32L for left eye and the right projection part 32R for right eye, and the left display part 33L for left eye and the right display part 33R for right eye have the symmetrical configurations to each other, and the configurations for the left eye will be explained below.

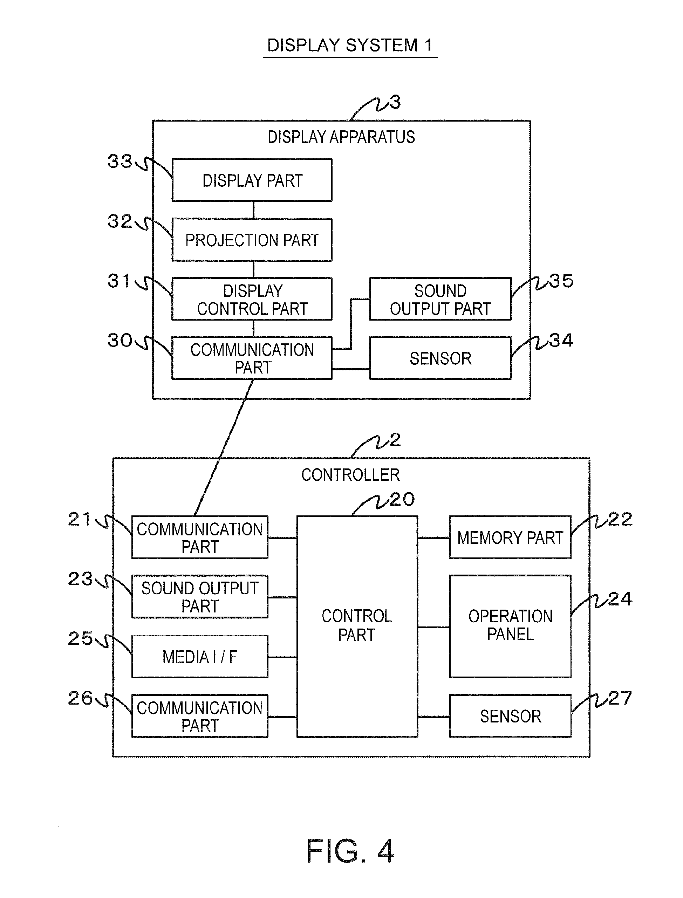

The left display control part 31L controls the left projection part 32L as will be described in detail with reference to Fig. 4. The left projection part 32L includes a left image generation part 320L and a left optical system 321L.

The left image generation part 320L includes a backlight source, a light modulation device, etc. The left image generation part 320L is controlled by the left display control part 31L.

The backlight source includes e.g. a collection of light sources with respect to each emission color such as red, green, and blue. As the light sources, e.g., light emitting diodes (LEDs) or the like may be used. The backlight source emits red light, green light, and blue light according to the control of the left display control part 31L and outputs the lights to the light modulation device.

The light modulation device is e.g. a liquid crystal display device as a display device. The light modulation device spatially modulates the red light, the green light, and the blue light output from the backlight source according to the control of the left display control part 31L, and thereby, outputs image lights in response to the image signals sent from the controller 2.

The left optical system 321L includes e.g. a group of projection lenses that project the incident image lights. The left optical system 321L projects the image lights output from the light modulation device into parallel luminous flux.

The left display part 33L includes a left light guide part 330L, a left reflection part 331L, and a left shade 332L.

The left light guide part 330L includes e.g. a light guide plate or the like. The left light guide part 330L guides the light projected from the left optical system 321L of the left projection part 32L to the left reflection part 331L along a predetermined optical path. That is, the part projects image light L1 formed to be parallel luminous flux by the left projection part 32L on a predetermined semi-transmissive reflection plane of a triangular prism of the left reflection part 331L, which will be described later.

The semi-transmissive reflection plane is formed in the left reflection part 331L, and a reflection coating such as a mirror layer is applied to the side that faces the left eye EL of the user when the display apparatus 3 is worn of the front and rear sides of the semi-transmissive reflection plane. The image light L1 transmitted by the left light guide part 330L and projected on the semi-transmissive reflection plane is totally reflected toward the left eye EL of the user by the surface with the reflection coating. Thereby, the reflected light enters the left eye EL of the user and forms a virtual image on the retina of the left eye EL.

Further, at least part of light L2 entering from the opposite surface to the left eye EL of the user (i.e., light from the outside world) of the front and rear sides of the semi-transmissive reflection plane of the left reflection part 331L is transmitted through the semi-transmissive reflection plane and guided to the left eye EL of the user. Thereby, the user can see a superimposition of an image in response to the image light L1 projected from the left projection part 32L and an optical image (outside world image) in response to the light L2 from the outside world.

The left shade 332L reduces the light L2 entering the left reflection part 331L from the outside world in response to its transmittance.

As described above, the user wears the display apparatus 3 on the head, and thereby, can see an image in response to the image light L1 output from the reflection parts 331 (left reflection part 331L, right reflection part 331R) as if the image were displayed and recognize the image. Further, the user can see the outside world with the display apparatus 3 worn on the head by at least part of the light L2 transmitted through the reflection parts 331 (left reflection part 331L, right reflection part 331R) from the outside world. That is, the display apparatus 3 allows the user to visually recognize an image generated based on image signals superimposed on an outside world image.

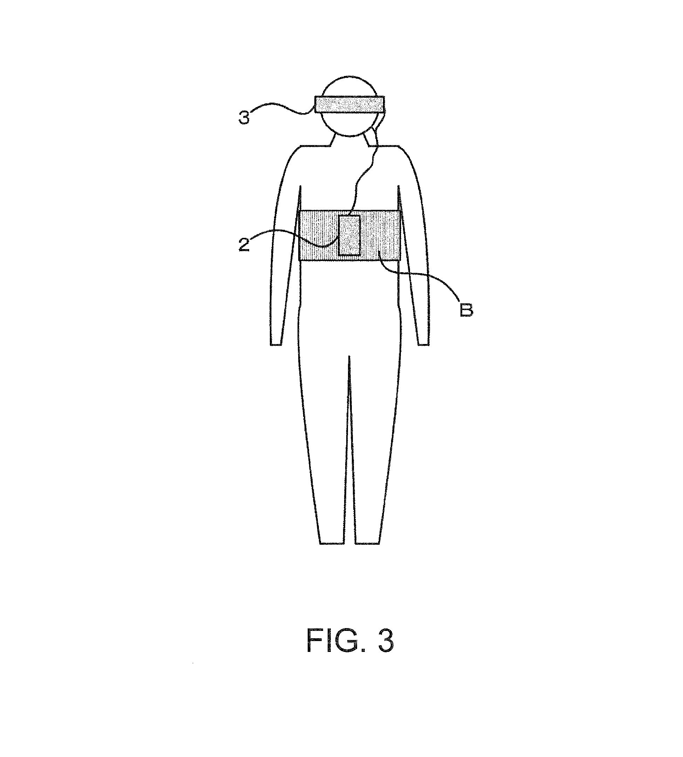

Fig. 3 is a diagram for explanation of an example of attachment locations of the display apparatus etc. to the user.

In the first embodiment, the display apparatus 3 is attached to the head to cover the eyes of the user. The controller 2 is attached to the torso of the user, for example, so that the controller 2 may take a predetermined position with respect to the user as will be described later in detail. The attachment method of the controller 2 is not particularly limited. For example, a band B may be wrapped around the torso of the user and the band and the controller 2 may be fastened by a hook-and-loop fastener or the like. In this regard, the controller 2 may be attached to be located at a height of the seventh cervical spine, the second thoracic spine, or the fourth or fifth lumbar spine, or the like. The height of the controller 2 may be appropriately adjusted in response to the symptom of the user. Note that, in the example of Fig. 3, the controller 2 is attached to the belly side of the user, however, may be attached to the back side.

Fig. 4 shows an example of a configuration of the display system.

The controller 2 has a control part 20, a communication part 21, a memory part 22, a sound output part 23, an operation panel 24, a media I/F (interface) 25, a communication part 26, and a sensor 27 (first sensor). Note that the control part 20 corresponds to a detection part and a generation part, the communication part 21 corresponds to a transmitting part and a receiving part, the communication part 26 corresponds to the transmitting part and the receiving part, the operation panel 24 corresponds to an input part, a display part, and an output part, the media I/F 25 corresponds to a reading part and a writing part, and the sound output part 23 corresponds to the output part.

The control part 20 includes e.g. a CPU (Central Processing Unit) and a RAM (Random Access Memory) used for temporary storage of various data etc., and performs integrated control of the operation of the controller 2 by operating according to control programs stored in the memory part 22.

For example, the control part 20 generates image signals to be output to the display apparatus 3 based on images of display objects of image data received via the communication part 26, image data read via the media I/F 25, image data read from the memory part 22, etc. The image signals contain e.g. image signals for left eye and image signals for right eye. The image signals for left eye and the image signals for right eye may be the same image signals or image signals differentiated in response to parallax of images.

Further, for example, the control part 20 generates sound signals to be output to the display apparatus 3 based on sound as output objects of sound data received via the communication part 26, sound data read via the media I/F 25, sound data read from the memory part 22, etc. The sound signals contain e.g. sound signals for left eye and sound signals for right eye.

Furthermore, in the embodiment, the control part 20 generates image signals of images representing the tilt of the torso of the user based on information output from the sensor 27, and outputs the signals to the display apparatus 3 via the communication part 21. This point will be described later in detail.

The communication part 21 is connected to the display apparatus 3 and communicates with the display apparatus 3. For example, the communication part 21 transmits image signals input from the control part 20 etc. to the display apparatus 3 and outputs signals received from the display apparatus 3 etc. to the control part 20 according to the instructions of the control part 20.

The memory part 22 includes e.g. a mask ROM (Read Only Memory) and a nonvolatile memory such as a flash memory or an FeRAM (Ferroelectric RAM). The control programs for control of the operation of the controller 2, various data, etc. are stored in the memory part 22.

The sound output part 23 has e.g. a speaker, a drive circuit for the speaker, a D/A converter, etc., and outputs sound based on the sound signals input from the control part 20 according to the instructions of the control part 20.

The operation panel 24 includes an input device for operation with respect to the controller 2, and a display that displays various kinds of information. The input device is realized by e.g. a capacitance touch panel, touch sensor, or the like. The display is e.g. a liquid crystal display, an organic EL (Electro-Luminescence) display, or the like. When the user operates the input device, the operation panel 24 outputs the operation signals according to the operation contents of the user to the control part 20 and also displays operation screens, messages, dialogs, etc. according to the image signals input from the control part 20.

The media I/F 25 is a device for reading and writing of data with respect to a portable memory medium such as a flash memory. For example, the media I/F 25 reads data from the memory medium and outputs the data to the control part 20 and writes data input from the control part 20 in the memory medium according to the instructions of the control part 20.

The communication part 26 is connected to a communication line of wireless LAN or wired LAN and performs communication with an external apparatus such as a PC (Personal Computer) or server. For example, the communication part 26 transmits information input from the control part 20 to the external apparatus and outputs information received from the external apparatus to the control part 20 according to the instructions of the control part 20. From the external apparatus, e.g. image contents and video contents are sent.

The sensor 27 includes e.g. one or more of a gyro sensor, an acceleration sensor, a geomagnetic sensor, a tilt sensor, a gravity sensor, etc. The sensor 27 outputs detected sensor information (information of angular acceleration, acceleration, direction, tilt, gravity, etc.) to the control part 20.

The display apparatus 3 has a communication part 30, the display control parts 31, the projection parts 32, the display parts 33, a sensor 34 (third sensor), and a sound output part 35. Note that the display part 33 corresponds to the output part, the sound output part 35 corresponds to the output part, and the communication part 30 corresponds to the transmitting part and the receiving part.

The communication part 30 is connected to the controller 2 and communicates with the controller 2. For example, the communication part 30 outputs the image signals received from the controller 2 to the display control parts 31 and outputs the sound signals received from the controller 2 to the sound output part 35. Further, for example, the communication part 30 transmits information output from the sensors 34 to the controller 2. Note that two of the communication parts 30 may be provided for left eye and right eye.

The display control parts 31 (left display control part 31L, right display control part 31R) control the projection parts 32 (left projection part 32L, right projection part 32R). The left display control part 31L controls the light modulation device of the left projection part 32L in response to the image signals for left eye received from the controller 2 via the communication part 30 to spatially modulate and output red light, green light, and blue light output from the backlight source. Further, for example, the left display control part 31L controls luminance of the backlight source and controls transmittance of the left shade 332L in response to the image signals for left eye received from the controller 2 via the communication part 30. For example, the right display control part 31R controls the light modulation device of the right projection part 32R in response to the image signals for right eye received from the controller 2 via the communication part 30 to spatially modulate and output red light, green light, and blue light output from the backlight source. Further, for example, the right display control part 31R controls luminance of the backlight source and controls transmittance of the right shade 332R in response to the image signals for right eye received from the controller 2 via the communication part 30.

The projection part 32 (left projection part 32L, right projection part 32R) and the display parts 33 (left display part 33L, right display part 33R) are as described above using Figs. 1 and 2.

The sensor 34 can include e.g. one or more of a gyro sensor, an acceleration sensor, a geomagnetic sensor, a tilt sensor, a gravity sensor, etc. The sensor 34 outputs detected sensor information (information of angular acceleration, acceleration, direction, tilt, gravity, etc.) to the communication part 30.

The sound output part 35 has e.g. a speaker, a drive circuit for the speaker, a D/A converter, etc., and outputs sound based on the sound signals input from the communication part 30 according to the instructions of the communication part 30.

Next, the characteristic operation of the display system 1, i.e., the operation of displaying the tilt of the torso of the user will be explained.

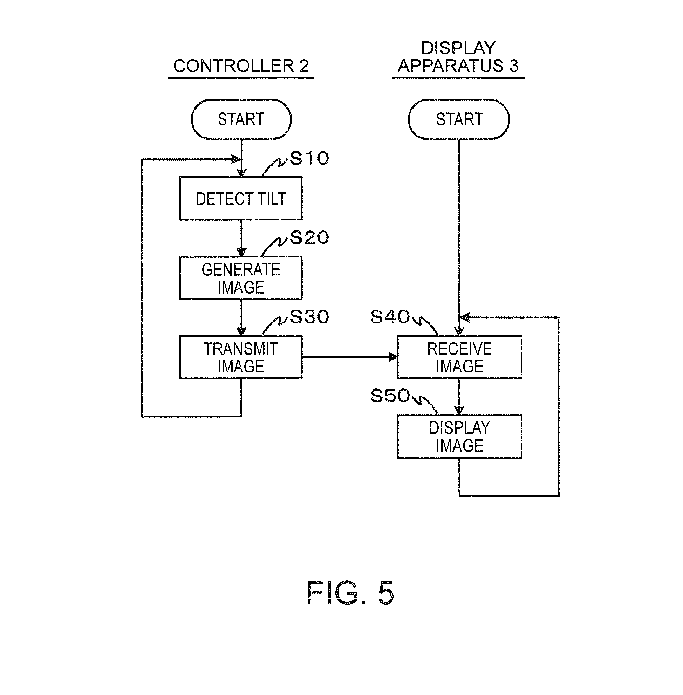

Fig. 5 is a flowchart showing an example of an operation of the display system. Note that the power of the controller 2 and the display apparatus 3 is turned on to be communicable with each other.

As a preparatory stage, for example, the caregiver attaches the display apparatus 3 to the head of the patient and attaches the controller 2 to the torso (belly side or back side) of the patient. For example, the controller 2 is attached to the torso of the patient to take a position such that the Y-axis of the controller 2 (see Fig. 1) may be along the body axis P of the patient (see Figs. 15A and 15B).

After the preparation, for example, when receiving an instruction to start a rehab support function from the caregiver or patient via the operation panel 24, the control part 20 starts processing of the flowchart shown in Fig. 5.

First, the controller 2 detects a tilt (step S10). Specifically, the control part 20 obtains the tilt of the controller 2 based on output from the sensor 27. The method of obtaining the tilt of the controller 2 is not particularly limited. For example, the control part 20 may acquire three angles of Azimuth about the Z-axis, Pitch about the X-axis, and Roll about the Y-axis, and obtain the tilt based on these angles. The part may obtain the tilt based on acceleration of the respective axes of X, Y, Z of the acceleration sensors.

In the embodiment, the control part 20 obtains the angle about the Z-axis of the controller 2 as the tilt of the controller 2. The controller 2 is attached to the torso of the patient as described above, and may use the angle about the Z-axis corresponding to the tilt of the torso of the patient. Note that, for example, the angle contains a direction from a reference angle (e.g., indicating the clockwise by plus and the counter-clockwise by minus).

Then, the controller 2 generates an image (step S20). Specifically, the control part 20 generates an image representing the tilt or a tilt obtained by making a predetermined correction to the tilt as the tilt of the torso of the patient based on the tilt detected at step S10. Note that, as the degree of the correction, it is preferable to generate an image having twice or more of the tilt detected at step S10, and more preferable to generate an image having twice the tilt.

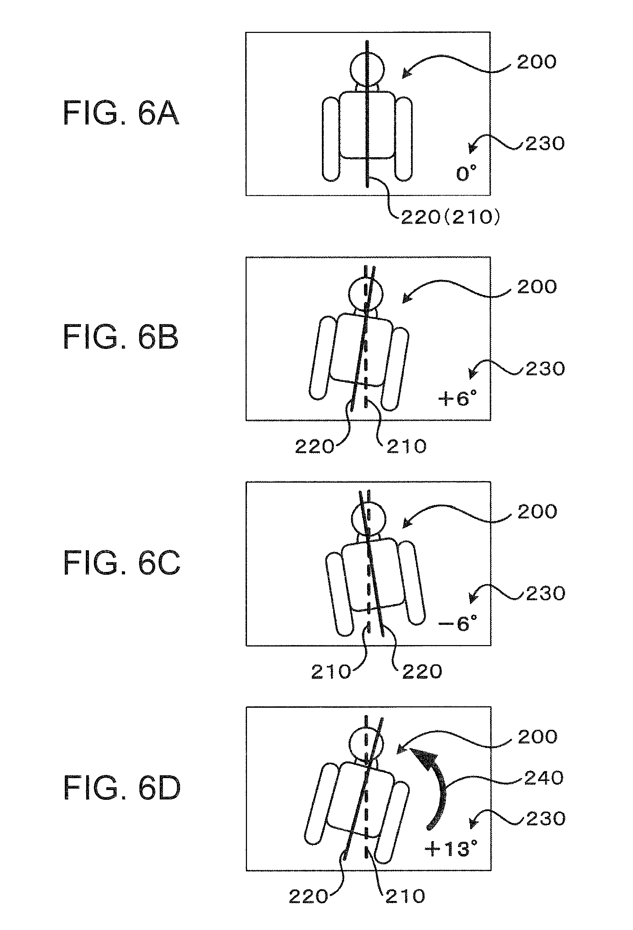

For example, as shown in Figs. 6A to 6D (showing examples of images representing tilts), the control part 20 generates images each containing a body image 200 representing the upper body as seen from the back side of the patient, an axis image 210 representing the normal body axis NP, an axis image 220 representing the body axis P of the patient, angle information 230 representing the angle of the tilt, a direction image 240 representing a direction in which the body axis P is brought closer to the body axis NP, etc. The axis image 210 is also an image representing the direction of gravity. Note that the upward and downward directions in Figs. 6A to 6D correspond to vertical directions and the leftward and rightward directions in Figs. 6A to 6D correspond to horizontal directions.

For example, the control part 20 statistically displays the head and the neck contained in the body image 200 in the median state (along the vertical direction), and dynamically displays the torso contained in the body image 200 in response to the detected tilt. Further, for example, the control part 20 statistically displays the axis image 210 in the median state (along the vertical direction), and dynamically displays the axis image 220 in response to the detected tilt. Furthermore, for example, the control part 20 displays the detected tilt in the angle information 230. The control part 20 determines whether or not the detected tilt is beyond a predetermined angle range (e.g., a range from -6 to +6 degrees) and, if the detected tilt is beyond the range, for example, displays an image showing an opposite direction to the detected tilt as the direction image 240. Note that, regardless whether or not the detected tilt is beyond the range, the direction image 240 may be displayed when the tilt is detected.

Fig. 6A shows an image when the body axis P of the patient is at the median. Fig. 6B shows an image when the body axis P of the patient tilts by six degrees in the rightward direction. Fig. 6C shows an image when the body axis P of the patient tilts by six degrees in the leftward direction. Fig. 6D shows an image when the body axis P of the patient tilts by 13 degrees in the rightward direction beyond the predetermined angle range.

The images shown in Figs. 6A to 6D are just examples and not limitations. For example, the head and neck contained in the body image 200 may be dynamically displayed in response to the detected tilt. Further, in addition to or in place of the axis image 210 representing the vertical directions, an axis image representing the horizontal directions may be statistically displayed.

Then, the controller 2 transmits the image (step S30). Specifically, the control part 20 generates image signals to be output to the display apparatus 3 based on the image generated at step S20 and transmits the signals to the display apparatus 3 via the communication part 21. Then, the control part 20 performs the processing at step S10 again.

The display apparatus 3 repeats processing at step S40 and step S50. Specifically, the communication part 30 receives the image signals transmitted from the controller 2 at step S30 and outputs the signals to the display control parts 31 (step S40). Further, the display control parts 31 control the projection parts 32 to display images on the display parts 33 based on the image signals output from the communication part 30 at step S40 (step S50).

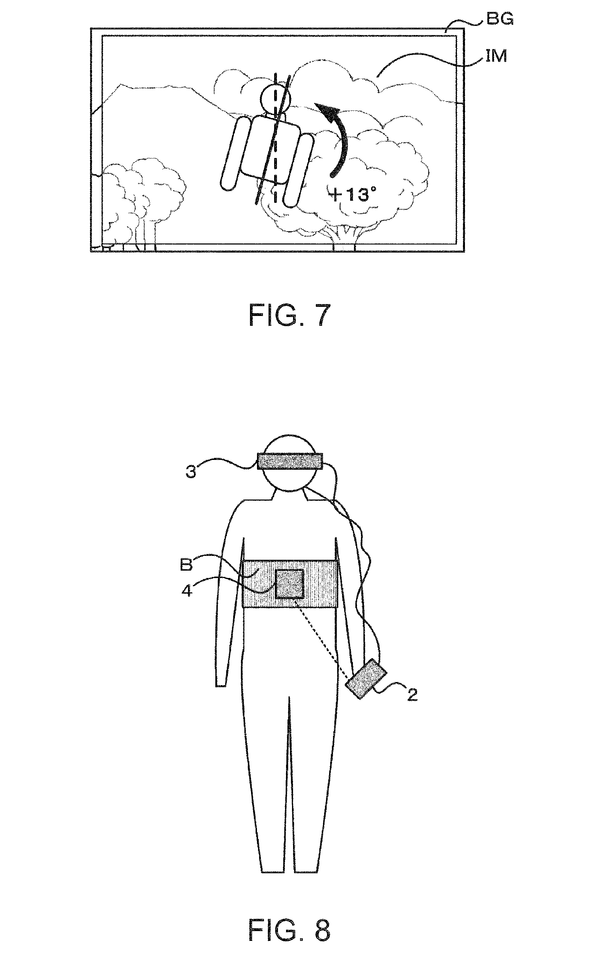

In this manner, for example, as shown in Fig. 7 (showing a screen example displayed on the display apparatus), an image IM representing the tilt of the torso of the patient generated at step S20 is displayed with an optical image BG of the outside world on the display parts 33. Thereby, the patient sees the image IM, and thereby, may recognize the body axis P of his or her own with a relative difference to the body axis NP. Further, the patient can seen the optical image BG of the outside world, and thereby, may recognize the body axis P with a relative difference to an object that can be treated as the vertical reference of the outside world (e.g. a desk, bed, or the like).

As above, the first embodiment of the invention is explained. According to the embodiment, the rehab of the patient having an abnormality in posture and balance may be supported to be performed more easily using the display apparatus. Further, in a location without equipment such as a mirror, the rehab may be easily performed. Furthermore, even when it is difficult for the patient to face the front, the rehab may be easily performed.

### Second Embodiment

In the first embodiment, the sensor 27 of the controller 2 is used as the device that detects the tilt of the user, however, in the second embodiment, a sensor 4 (second sensor) separate from the controller 2 is used. Below, the differences from the first embodiment will be explained.

Fig. 8 is a diagram for explanation of an example of attachment locations of a display apparatus etc. according to the second embodiment of the invention to a user.

In the second embodiment, the sensor 4 is communicably connected to the controller 2. Further, for example, the sensor 4 is attached to the torso of the user to take a predetermined position with respect to the user. The attachment method of the sensor 4 is not particularly limited. For example, a band B may be wrapped around the torso of the user and the band and the sensor 4 are fastened by a hook-and-loop fastener or the like. In this regard, the sensor 4 may be attached to be located at a height of the seventh cervical spine, the second thoracic spine, or the fourth or fifth lumbar spine, or the like of the user. The height of the sensor 4 may be appropriately adjusted in response to the symptom of the user. Note that, in the example of Fig. 8, the sensor 4 is attached to the belly side, however, may be attached to the back side or attached to the neck or arm of the user. For example, the controller 2 may be fixed to the band B or held in the hand of the user.

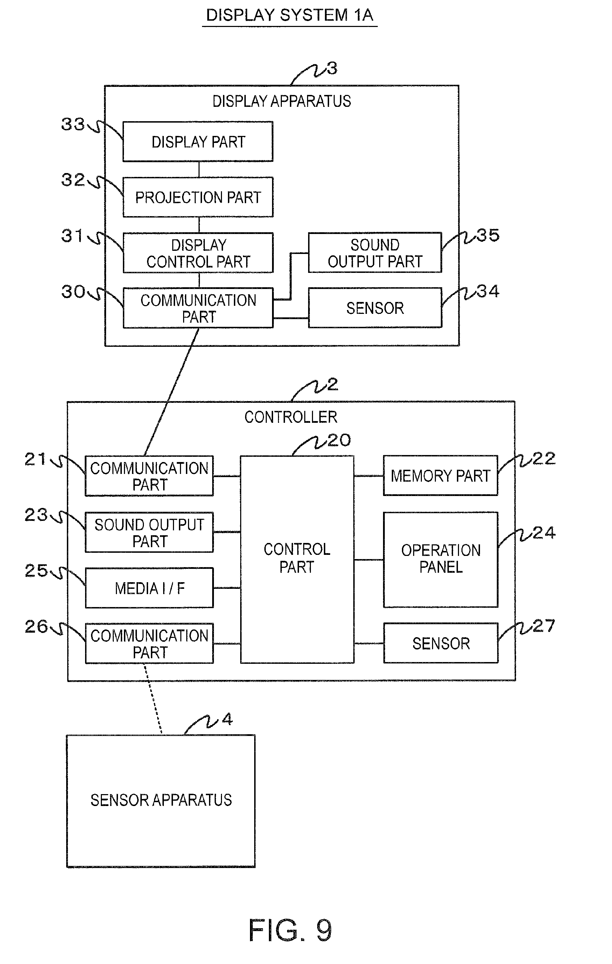

Fig. 9 shows an example of a configuration of a display system. A display system 1A has the controller 2, the display apparatus 3, and the sensor 4. In the second embodiment, the controller 2 and the sensor 4 are communicably connected. Note that, like the first embodiment, the controller 2 and the display apparatus 3 are communicably connected.

The sensor 4 has e.g. one or more sensors (not shown) of a gyro sensor, an acceleration sensor, a geomagnetic sensor, a tilt sensor, a gravity sensor, etc. and a communication part (not shown) that communicates with an external apparatus including the controller 2. The sensor 4 outputs sensor information (information of angular acceleration, acceleration, direction, tilt, gravity, etc.) detected by the sensor to the controller 2 via the communication part. Note that the communication part corresponds to the transmitting part.

The communication part 26 of the controller 2 communicates with the sensor 4. The control part 20 receives information output from the sensor 4 via the communication part 26, generates image signals of an image representing the tilt of the torso of the user based on the received information, and outputs the signals to the display apparatus 3 via the communication part 21.

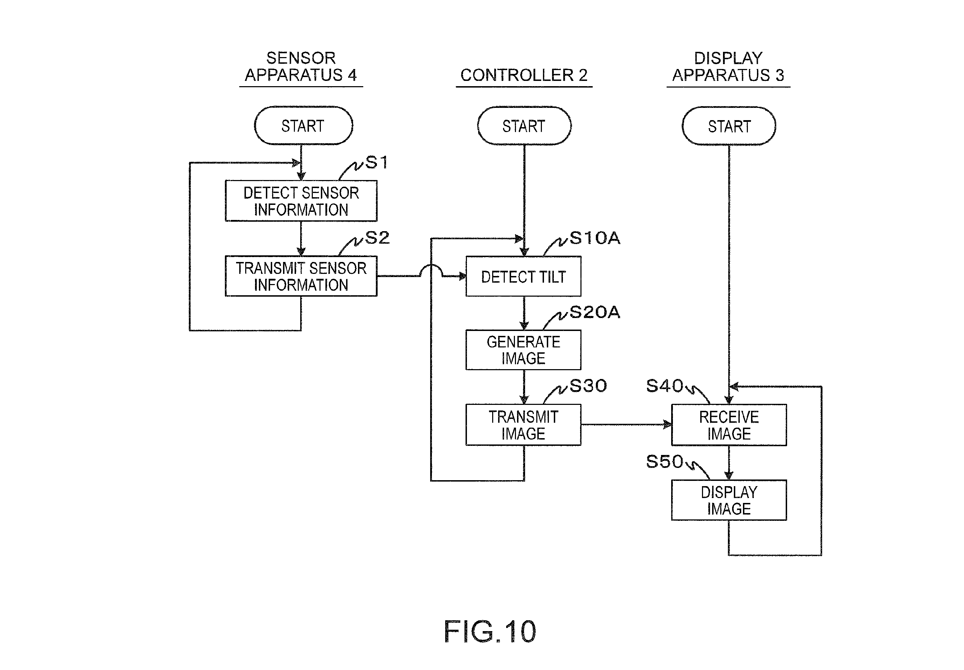

Fig. 10 is a flowchart showing an example of an operation of the display system. Note that the power of the controller 2, the display apparatus 3, and the sensor 4 is turned on to be communicable with one another.

As a preparatory stage, for example, the caregiver attaches the display apparatus 3 to the head of the patient and attaches the sensor 4 to the torso (belly side or back side) of the patient. The controller 2 may be attached to the torso (belly side or back side) of the patient or held in the hand of the patient or caregiver. For example, the sensor 4 is attached to the torso of the patient to take a position such that the Y-axis of the sensor 4 may be along the body axis P of the patient (see Figs. 15A and 15B). Here, the sensor 4 has X, Y, Z axes like the controller 2.

After the preparation, for example, when receiving an instruction to start a rehab support function from the caregiver or patient via the operation panel 24, the control part 20 starts processing of the flowchart shown in Fig. 10. The processing at steps S30, S40, S50 shown in Fig. 10 is the same as the processing at steps S30, S40, S50 shown in Fig. 5 and the explanation will be omitted.

The sensor 4 repeats processing at step S1 and step S2. Specifically, the sensor 4 detects sensor information by a sensor inside (step S1). Further, the sensor 4 transmits the sensor information detected at step S1 to the controller 2 via the communication part (step S2). Then, the sensor 4 performs the processing at step S1 again.

On the other hand, the controller 2 detects a tilt (step S10A). Specifically, the control part 20 receives the sensor information transmitted from the sensor 4 at step S2 via the communication part 26 and obtains the tilt based on the received information. The method of obtaining the tilt of the sensor 4 is the same as that of obtaining the tilt of the controller 2. Then, the controller 2 generates an image (step S20A). Specifically, the control part 20 generates an image representing the tilt obtained at step S10A as the tilt of the torso of the patient. The method of generating the image is the same as that at step S20 in Fig. 5 and the explanation will be omitted.

As above, the second embodiment of the invention is explained. According to the second embodiment, the same advantages as those of the first embodiment may be obtained. Further, in the second embodiment, it is unnecessary to fix the controller 2 to the torso while the tilt is displayed and it is easier for the caregiver or patient to hold and operate the controller 2. Furthermore, in the second embodiment, the sensor 4 is a separate apparatus from the controller 2, and detection accuracy of the tilt may be easily made higher only by replacement of the sensor 4 by another product. In addition, when the controller 2 and the display apparatus 3 are wirelessly connected, the controller 2 may be held by the caregiver and the rehab may be easier.

Note that, in the first embodiment and the second embodiment, the image representing the tilt of the patient is generated in the controller 2, however, not limited to that. For example, the controller 2 may transmit the information representing the obtained tilt to the display apparatus 3 and the display apparatus 3 may generate the image representing the tilt of the patient based on the information representing the tilt.

### Third Embodiment

In the second embodiment, the sensor 4 and the controller 2 communicate with each other, however, in the third embodiment, an information processing apparatus 5 such as a PC is provided, the sensor 4 and the information processing apparatus 5 communicate with each other, and the information processing apparatus 5 and the controller 2 communicate with each other. Below, the differences from the second embodiment will be explained.

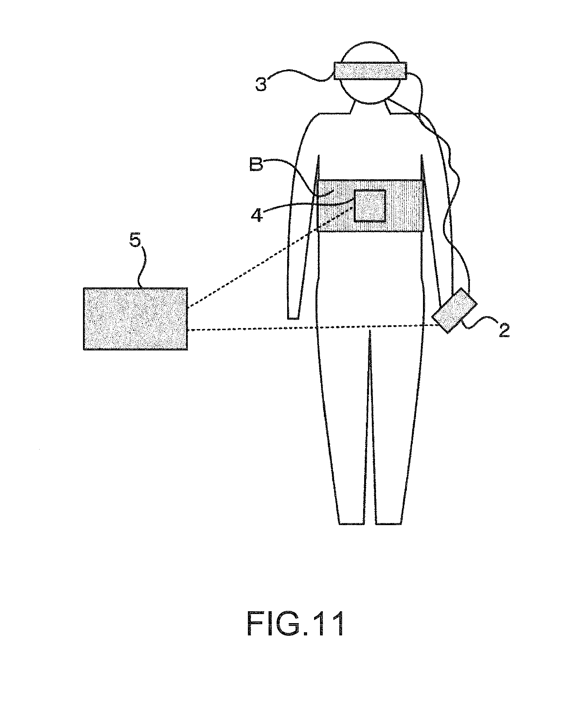

Fig. 11 is a diagram for explanation of an example of attachment locations of a display apparatus etc. according to the third embodiment of the invention to a user.

In the third embodiment, the sensor 4 is communicably connected to the controller 2. Further, for example, the sensor 4 is attached to the torso of the user to take a predetermined position with respect to the user. Furthermore, for example, the controller 2 may be fixed to the band B or held in the hand of the user. For example, the information processing apparatus 5 is installed in a location apart from the patient and communicably connected to the sensor 4 and the controller 2.

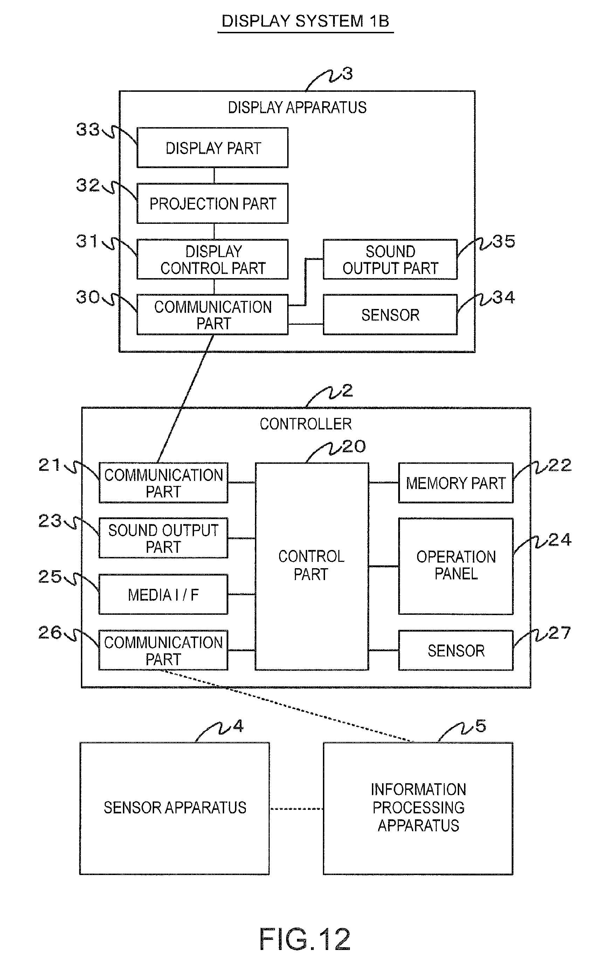

Fig. 12 shows an example of a configuration of a display system. A display system 1B has the controller 2, the display apparatus 3, the sensor 4, and the information processing apparatus 5. In the third embodiment, the information processing apparatus 5 is communicably connected to the sensor 4 and the controller 2. Note that, like the second embodiment, the controller 2 and the display apparatus 3 are communicably connected.

The sensor 4 transmits sensor information detected by a sensor (information of angular acceleration, acceleration, direction, tilt, gravity, etc.) to the information processing apparatus 5 via a communication part (not shown).

The information processing apparatus 5 has e.g. a control part, a communication part, etc. (not shown). The communication part of the information processing apparatus 5 communicates with the sensor 4. The control part of the information processing apparatus 5 receives sensor information output from the sensor 4 via the communication part, generates an image representing the tilt of the torso of the user based on the received sensor information, and transmits the image to the controller 2 via the communication part. The image generation processing is the same as that of the second embodiment. Note that the control part corresponds to the detection part and the communication part corresponds to the transmitting part and the receiving part.

The communication part 26 of the controller 2 receives the image representing the tilt of the torso of the user from the information processing apparatus 5. The control part 20 generates image signals based on the received image, and outputs the signals to the display apparatus 3 via the communication part 21.

As above, the third embodiment of the invention is explained. According to the third embodiment, the same advantages as those of the first embodiment may be obtained. Further, according to the third embodiment, the same advantages as those of the second embodiment may be obtained. Furthermore, in the third embodiment, generation of the image is performed in the information processing apparatus 5, and load of image processing in the controller 2 may be reduced and other processing is easily performed in the controller 2.

Note that the information processing apparatus 5 may generate image signals based on the generated image and transmit the image signals to the controller 2. In this case, the controller 2 may transfer the received image signals to the display apparatus 3.

### First Modified Example

In the first to third embodiments, the single sensor contained in the controller 2 or sensor 4 is used however, in the modified example, a plurality of sensors may be used. Here, the case where two sensors 4 (the second sensor) are used in the second embodiment is taken as an example, and differences from the second embodiment will be explained.

For example, the caregiver attaches one of the sensors 4 to the vicinity of the lumbar spine of the patient and attaches the other sensor 4 to the vicinity of the cervical spine of the patient. These second sensors 4 may respectively transmit information representing the tilt to the controller 2.

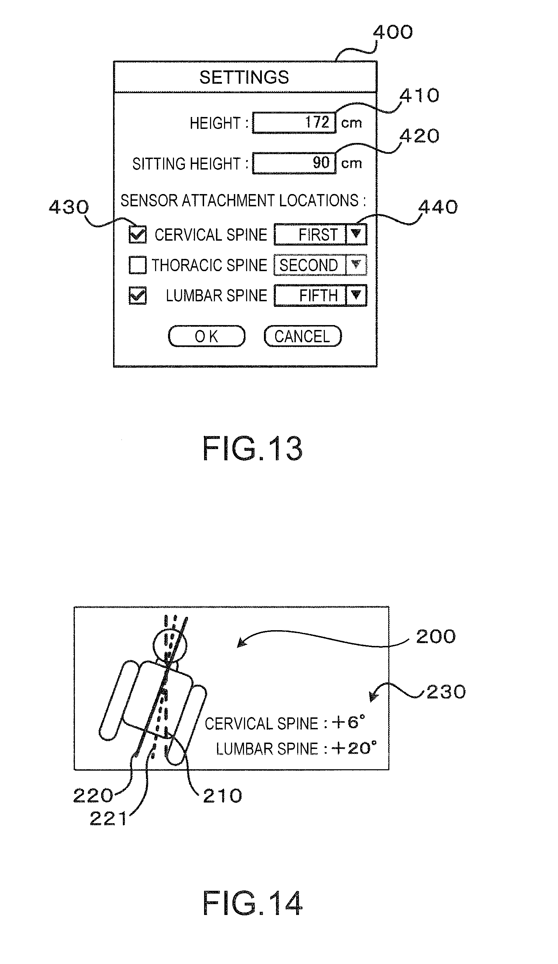

For example, the control part 20 of the controller 2 allows the operation panel 24 to display an operation screen 400 as shown in Fig. 13 (showing an example of an operation screen according to the first modified example). On the operation screen 400, a height field 410 for entry of the height of the patient, a sitting height field 420 for entry of the sitting height of the patient, fields 430 for entry of attachment or non-attachment of sensors to the patient and their locations, and fields 440 for entry of the detailed locations of the sensors set in the fields 430 are displayed. The fields 430 and 440 are provided for each sensor and, in the example of the drawing, they correspond to the one of the sensors 4 and the other sensor 4 sequentially from the top. Further, the fields 430 and 440 are provided in the order of the heights of the locations in the body axis of the corresponding sensors (the order of the cervical spine, the thoracic spine, the lumbar spine). The respective fields 430 are e.g. check boxes and one or more sensors may be selected. The fields 440 are e.g. drop-down lists and the locations may be selected among a plurality of options.

The control part 20 receives various kinds of settings in the operation screen 400 via the operation panel 24. That is, the control part 20 may acquire the height and the sitting height of the patient and the attachment locations of one or more sensors via the operation screen 400. In the example of Fig. 13, the height "172 cm", the sitting height "90 cm", the location of the one of the sensors 4 "first cervical spine" , the location of the other sensor 4 "fifth lumbar spine" may be acquired.

When the rehab support function is executed, the control part 20 receives information output from the two sensors 4 via the communication part 26, and generates image signals of the image representing the tilt of the user based on the received information.

Here, for example, as shown in Fig. 14 (showing an example of the image representing the tilt), the control part 20 displays an axis image 221 representing the axis of the head and the neck of the patient in addition to the axis image 220 representing the body axis P of the patient. The axis of the head and the neck corresponds to the tilt detected by the one of the sensors 4 and the body axis P corresponds to the tilt detected by the other sensor 4.

For example, the control part 20 dynamically displays the head and the neck contained in the body image 200 in response to the tilt detected based on the information output from the one of the sensors 4, and dynamically displays the torso contained in the body image 200 in response to the tilt detected based on the information output from the other sensor 4. Further, for example, the control part 20 statistically displays the axis image 210 in the median state (along the vertical direction), dynamically displays the axis image 220 in response to the tilt detected by the other sensor 4, and dynamically displays the axis image 221 in response to the tilt detected by the one of the sensors 4. Furthermore, for example, the control part 20 displays the tilt detected by the one of the sensors 4 and the tilt detected by the other sensor 4 in the angle information 230. The control part 20 may determine whether or not the detected tilt is beyond a predetermined angle range (e.g. , a range from -6 to +6 degrees) and, if the detected tilt is beyond the range, for example, display an image showing an opposite direction to the detected tilt as the direction image 240.

The control part 20 generates image signals to be output to the display apparatus 3 based on the image generated in the above described manner, and transmits the signals to the display apparatus 3 via the communication part 21.

As above, the first modified example of the respective embodiments of the invention is explained. According to the first modified example, the image representing the tilts of the plurality of locations of the upper body of the user are displayed based on the tilts acquired from the plurality of sensors, and thereby, the user may recognize the posture of him- or herself more easily and more accurately.

In the first modified example, the two sensors 4 are used as the plurality of sensors, however, not limited to the configuration. For example, the sensor 27 of the controller 2 and one or more sensors 4 may be used. In this case, the controller 2 generates an image representing the tilt detected by the sensor 27 and the tilts detected by the one or more sensor 4 as tilts of the respectively corresponding locations of the body of the patient. For example, three or more sensors 4 may be used.

Note that, in the case where the first modified example is applied to the third embodiment, the information processing apparatus 5 may detect the tilts by a plurality of sensors 4 or detect the tilts by the controller 2 and one or more sensors 4 , and generate an image representing the detected tilts as tilts of the respectively corresponding locations of the body of the patient.

### Other Modified Examples

In the above described embodiments and modified example, the tilt is detected by the sensor 27 (the first sensor) of the controller 2 and the sensor 4 (the second sensor), however, the tilt may be detected by the sensor 34 (the third sensor) of the display apparatus 3. In this case, the communication part 30 transmits sensor information output from the sensor 34 to the controller 2. The control part 20 receives the sensor information via the communication part 21 and obtains the tilt based on the sensor information.

In the above described embodiments and modified example, the image representing the tilt is displayed on the display apparatus 3, however, the image may be displayed on another display in addition to the display apparatus 3. For example, the control part 20 outputs the generated image to the operation panel 24 for display. Further, for example, the information processing apparatus 5 outputs the generated image to a display that the apparatus has or a display connected to the apparatus.

In the above described embodiments and modified example, the control part 20 of the controller 2 may chronologically record the tilts acquired by the sensor in the memory part 22 or the like. In this case, the control part 20 may generate an image of a graph or the like representing the tilt changes based on the recorded information and output the image to the operation panel 24 or the display apparatus 3. Further, the information processing apparatus 5 may also chronologically record the tilts acquired by the sensor in the memory part 22 or the like, generate an image of a graph or the like representing the tilt changes based on the recorded information, and output the image to the display.

In the above described embodiments and modified example, for example, as shown in Fig. 6B, the image is generated so that the body image 200, the axis image 210, the axis image 220, and the angle information 230 may be displayed, however, not limited to the configuration. For example, it is only necessary that the image is generated so that at least the axis image 220 or the body image 200 may be displayed. The image may be generated so that a lattice-patterned image may be displayed in addition to or in place of the body image 200. In these cases, the user may recognize the tilt of him- or herself based on the axis image 220, the body image 200, or the lattice-patterned image and the image of the outer world transmitted and displayed.

In the above described embodiments and modified example, if the detected tilt is beyond the predetermined angle range, the direction image 240 is displayed, however, in addition to or in place of the display, the control part 20 may output alarm sound via the sound output part 23 if the detected tilt is beyond the predetermined angle range. The control part 20 may transmit a sound signal of the alarm sound to the display apparatus 3 via the communication part 21, and the communication part 30 may output the alarm sound based on the sound signal received from the controller 2 via the sound output part 35. It is only necessary that at least one of the sound output part 23 or the sound output part 35 outputs the alarm. Thereby, the rehab may be supported while the attention is drawn.

Note that the configurations including the shapes, sizes, arrangements of parts of the display apparatus 3 and the controller 2 explained in Figs. 1 and 2 are not limited to those shown in the drawings as long as the above described advantages of the invention may be obtained.

Further, the configurations of the display systems 1, 1A, 1B shown in Figs. 4, 9, 12, etc. are formed by classification according to the main processing details for facilitation of understanding of the configurations of the display systems 1, 1A, 1B. The invention is not limited by the way and names of classification of the component elements. The configurations of the display systems 1, 1A, 1B may be classified into more component elements according to the processing details. Further, one component element may be classified to execute more pieces of processing. The processing of the respective component elements may be executed by a single piece of hardware or a plurality of pieces of hardware.

The assignation of the processing or functions of the respective apparatuses including the controller 2 contained in the display systems 1, 1A, 1B are not limited to those described above as long as the advantage of the invention may be achieved. For example, at least a partial function of the controller 2, e.g. the function of generating the image based on the sensor information may be included in the display apparatus 3. In this case, the display control part 31 of the display apparatus 3 generates image signals of the image representing the tilt based on the sensor information output by at least one sensor of the sensor 27 of the controller 2 and the sensors of one or more sensors 4, and performs display based on the image signals.

The units of processing in the flowcharts shown in Fig. 5 etc. are formed by division according to the main processing details for facilitation of understanding of the configurations of the display systems 1, 1A, 1B. The invention is not limited by the way and names of division of the units of processing. The processing of the display systems 1, 1A, 1B may be divided into more units of processing according to the processing details. Further, one unit of component may be divided to contain more pieces of processing. The processing sequences of the flowcharts are not limited to the examples shown in the drawings.

The invention may be applied to displays of tilts of the other respective parts in addition to the torso, head, or neck. Further, the invention may be used not only for the rehab of the pusher syndrome or the like but also for correction of postures and balances. Furthermore, the invention may be used not only in the upright position of the patient but also in the seated position and during walking. In addition, the invention may be applied not only to the so-called spectacle-shaped transmissive head mounted display but also to a display apparatus that can display images when attached to the head as long as the axis image 220, the body image 200, or the lattice-patterned image and the image of the outside world are displayed.

The entire disclosure of Japanese Patent Application No. 2014-110947 filed May 29, 2014 is expressly incorporated by reference herein.

## Claims

1. A display system comprising:
a display apparatus configured to be attached to a head of a user; and
a display control apparatus operatively connected to the display apparatus,
the display control apparatus including:
a detector configured to detect a tilted posture of the user, and
a transmitter configured to transmit a first tilt image representing the tilted posture to the display apparatus, and
the display apparatus including:
a receiver configured to receive the first tilt image from the display control apparatus, and
a display configured to display the first tilt image.

2. The display system according to claim 1, wherein the display control apparatus further includes a first sensor configured to detect the tilted posture, the first sensor being selectively attachable to the user.

3. The display system according to claim 2, further comprising a second sensor operatively connected to the display control apparatus,
wherein the second sensor is selectively attachable to the user,
the detector is configured to detect tilting of one or more parts of the user by the first sensor and the second sensor,
the transmitter is configured to transmit a second tilt image representing the tilting of the one or more parts to the display apparatus,
the receiver is configured to receive the second tilt image from the display control apparatus, and
the display is configured to display the second tilt image.

4. The display system according to claim 1, further comprising a second sensor operatively connected to the display control apparatus,
wherein the second sensor is selectively attachable to the user,
the detector is configured to detect tilting of one or more parts of the user by the second sensor,
the transmitter is configured to transmit another tilt image representing the tilting of the one or more parts to the display apparatus,
the receiver is configured to receive the another tilt image from the display control apparatus, and
the display is configured to display the another tilt image.

5. The display system according to claim 1, wherein the first tilt image includes an image representing a tilted torso of the user.

6. A display control apparatus connected to a display apparatus configured to be attached to a head of a user, the display control apparatus comprising:
a detector configured to detect a tilted posture of the user; and
a transmitter configured to transmit a first tilt image representing the tilted posture to the display apparatus.

7. The display control apparatus according to claim 6, wherein the first tilt image includes an image representing a tilted torso of the user.

8. The display control apparatus according to claim 6, wherein the detector is configured to detect the tilted posture of the user on the basis of information outputted from a sensor attached to the user.

9. A program for a display control apparatus connected to a sensor configured to detect a tilt and a display apparatus configured to be attached to a head of a user, allowing the display control apparatus to execute:
detecting a tilted posture of the user on the basis of an information outputted from the sensor;
generating a first tilt image representing the tilted posture; and
outputting the first tilt image to the display apparatus.
